# EUROPÄISCHE PATENTSCHRIFT

(11) **EP 1 408 735 B1**
(45) Veröffentlichungstag und Bekanntmachung des Hinweises auf die Patenterteilung: **14.04.2010**
(21) Anmeldenummer: 02762392.5
(22) Anmeldetag: 24.07.2002
(51) Int. Cl.: A01H 1/02, A01H 5/10, C12Q 1/68

(54) **MÄNNLICHE STERILITÄT IN GRÄSERN DER GATTUNG LOLIUM**
MALE STERILITY IN GRASSES OF THE GENUS LOLIUM
STERILITE MALE DANS DES GRAMINEES DU GENRE LOLIUM

(30) Priorität: 26.07.2001 DE 10136378
(43) Veröffentlichungstag der Anmeldung: 21.04.2004
(73) Patentinhaber: NORDDEUTSCHE PFLANZENZUCHT HANS-GEORG LEMBKE KG, 24363 Holtsee (DE)
(72) Erfinder: GAUE, Inge, 23999 Kaltenhof (DE); BAUDIS, Heinrich, 23999 Malchow (DE)
(74) Vertreter: Neuefeind, Regina
(86) Internationale Anmeldenummer: PCT/EP2002/008252
(87) Internationale Veröffentlichungsnummer: WO 2003/026392

(56) Entgegenhaltungen:
- US-A- 5 955 648
- US-A- 6 166 306
- WIT F: "CYTOPLASMIC MALE STERILITY IN RYE GRASSES LOLIUM-SPP DETECTED AFTER INTERGENERIC HYBRIDIZATION" EUPHYTICA, Bd. 23, Nr. 1, 1974, Seiten 31-38, XP009003230 ISSN: 0014-2336 in der Anmeldung erwähnt
- CONNOLLY V ET AL: "INDUCTION OF CYTOPLASMIC MALE-STERILITY INTO RYEGRASS LOLIUM-PERENNE" THEORETICAL AND APPLIED GENETICS, Bd. 68, Nr. 5, 1984, Seiten 449-453, XP009003245 ISSN: 0040-5752 in der Anmeldung erwähnt
- GAUE I.: "Ergebnisse von Untersuchungen zur Hybridzüchtung bei Lolium perenne" TAGUNGSBERICHT DER AKADEMIE DER LANDWITSCH.-WISS. , Bd. 191, 1981, Seiten 119-126, XP001120802 Berlin, DDR in der Anmeldung erwähnt
- KIANG A -S ET AL: "Cytoplasmic male sterility (CMS) in Lolium perenne L.: 1. Development of a diagnostic probe for the male-sterile cytoplasm." THEORETICAL AND APPLIED GENETICS, Bd. 86, Nr. 6, 1993, Seiten 781-787, XP009002836 ISSN: 0040-5752
- ROUWENDAL G J A ET AL: "MOLECULAR ASPECTS OF CYTOPLASMIC MALE STERILITY IN PERENNIAL RYEGRASS LOLIUM-PERENNE L. MTDNA AND RNA DIFFERENCES BETWEEN PLANTS WITH MALE-STERILE AND FERTILE CYTOPLASM AND RESTRICTION MAPPING OF THEIR ATP6 AND COXI HOMOLOGOUS REGIONS" THEORETICAL AND APPLIED GENETICS, Bd. 83, Nr. 3, 1992, Seiten 330-336, XP009002838 ISSN: 0040-5752
- FUJIMORI, M. ET AL: "Molecular analyses of cytoplasmic male sterility in Italian ryegrass ( Lolium multiflorum Lam.)" UTILIZATION OF TRANSGENIC PLANT AND GENOME ANALYSIS IN FORAGE CROPS. PROCEEDINGS OF AN INTERNATIONAL WORKSHOP HELD AT THE NATIONAL GRASSLAND RESEARCH INSTITUTE, NISHINASUNO, TOCHIGI, JAPAN, 17-18 MARCH 1998, (1998) PP. 161-166. NGRI WORKING REPORT NO, XP001121099
- SATO M ET AL: "Mitochondrial DNA analysis reveals cytoplasmic variation within a single cultivar of perennial ryegrass (Lolium perenne L.)." EUPHYTICA, Bd. 83, Nr. 3, 1995, Seiten 205-208, XP009002811 ISSN: 0014-2336

## Beschreibung

Die Erfindung betrifft ein Verfahren zur Herstellung von stabil männlich sterilen Pflanzen der Gattung *Lolium* zur Verwendung in der gezielten Produktion von Hybridsorten unter Ausnutzung von Heterosis.

Pflanzen besitzen als Eukaryonten zwei oder mehr Kopien ihrer genetischen Information pro Zelle. Jedes Gen wird in der Regel durch zwei Allele, die im homozygoten Zustand identisch bzw. im heterozygoten Zustand unterschiedlich sein können, repräsentiert. Bei der Kreuzung von zwei ausgewählten Inzuchtlinien sind die in der ersten Generation gebildeten F1-Hybriden, also heterozygote Individuen, häufig größer, robuster und auch ertragreicher als die homozygoten Eltern, vermutlich weil ihre beiden allelischen Genprodukte a) mit geringerer Wahrscheinlichkeit inaktiviert werden oder b) eine größere Reaktionsbreite aufweisen. Dieser als Heterosis oder Hybridvitalität bezeichnete Effekt wird bereits seit vielen Jahrzehnten von Pflanzenzüchtern zur Produktion von Hybrid-Sorten genutzt.

Unter dem Phänomen der Heterosis versteht man somit die Steigerung von quantitativen Merkmalsausprägungen in einer Nachkommenschaft über das Elternmittel bzw. über die Leistung des besseren Elters hinaus. Insbesondere die Wüchsigkeit (Pflanzenlänge, Verzweigungsgrad etc.) und der Ertrag sowie quantitativ vererbte Merkmale (u.a. Resistenzen) können hierbei betroffen sein.

Die Züchtung von Hybridlinien erfolgt unter Nutzung der cytoplasmatischen männlichen Sterilität (CMS) oder der Selbst-Inkompatibilität (SI), den beiden wichtigsten genetischen Systemen zur Verhinderung der Selbstbefruchtung. Eine vollständige Nutzung der Heterosis lässt sich durch die Ausnutzung der cytoplasmatisch vererbbaren männlichen Sterilität erreichen (C. Bothe, Nutzung von teilfertilen ms-Linien für die Züchtung von Chance-Hybriden bei Welschem Weidelgras (Lolium multiflorum Lam.), Dissertation Göttingen, Cuvillier Verlag Göttingen 1996, 113 S.; G. Kobabe, Heterosis and hybrid seed production in fodder grass, Monographs on Theoretical and Applied Genetics, Vol. 6 Heterosis, Hrsg.: R Frankel, Springer-Verlag Berlin Heidelberg 1983; V. Lein, Heterosis in Kreuzungen zwischen Inzuchtlinien des Deutschen und Welschen Weidelgrases (Lolium perenne L. x Lolium multiflorum Lam. ssp. italicum) Dissertation Göttingen 1988; 91 S.). Durch den Verlust der Fähigkeit eines Kreuzungspartners fertilen Pollen zu bilden, ermöglicht die cytoplasmatische männliche Sterilität eine gezielte Produktion von F₁-Hybriden. Die weibliche Fertilität ist davon nicht betroffen.

In der Züchtung von Kulturpflanzen mit verbesserten agronomischen Leistungen und angepassten Inhaltsstoffen lassen sich in Abhängigkeit des natürlichen Befruchtungsmodus der jeweiligen Pflanzenart unterschiedliche Zuchtmethoden anwenden. Während bei den strengen Selbstbefruchtern wie z.B. Gerste (*Hordeum vulgare* L.) und Weizen (*Triticum aestivum* L.) Methoden der Linienzüchtung zur Anwendung kommen, wurde bei obligaten Fremdbefruchtern wie dem Roggen (*Secale cereale* L.) in der Vergangenheit Populations-, Synthetic- und Hybridzüchtung angewendet, bei denen in zunehmendem Maße Heterosis genutzt wird.

Das für die Züchtung vorhandene Pflanzenmaterial weist eine natürliche Variabilität in der Ausprägung der Heterosis auf. Hierbei unterscheidet man die allgemeine Kombinationseignung (general combining ability, GCA) und die spezielle Kombinationseignung (specific combining ability, SCA). Linien mit einer hohen GCA zeichnen sich durch eine hohe heterotische Leistung in Kreuzungen mit unterschiedlichen Elternlinien aus. Pflanzenlinien mit einer hohen SCA weisen eine hohe heterotische Leistung in Kombination mit einem speziellen Kreuzungspartner auf. Die SCA kann somit nur in vergleichenden paarweisen Kreuzungen (z.B. Diallel) ermittelt werden.

Für die züchterische Arbeit im Hinblick auf die Nutzung von Heterosis wird i.d.R. Pflanzenmaterial selektiert, welches sich durch eine gute Eigenleistung und eine gute GCA bzw. SCA auszeichnet.

Während bei der Züchtung von Populationssorten zunächst Massenauslese, d.h wiederholte Auslese der besten Einzelpflanzen und gemeinsame Weiterführung zu einer homogenen Sorte, betrieben wird, werden für die Erzeugung sog. synthetischer Sorten (Synthetics) mehrere Linien, die sich durch eine gute GCA ausweisen, selektiert. Zur Saatguterzeugung werden dann gezielt verschiedene Linien gemeinsam über zwei bis drei Generationen bis zur Vermarktung als Saatgut angebaut. Diese Synthetics weisen i.d.R. ein höheres Maß an Heterosis und somit insbesondere eine höhere Ertragsleistung als die Populationssorten auf.

Hybridsorten stellen eine weitere Steigerung bei der Nutzung von Heterosis in Pflanzensorten dar. Durch die gezielte Kombination von bestimmten Elternlinien mit guter GCA bzw. SCA im letzten Schritt der Saatguterzeugung können Hybridsorten erzeugt werden, die sich durch eine sehr hohe Heterosisleistung und somit durch ein deutlich gesteigertes Ertragspotential auszeichnen.

Voraussetzung zur Erzeugung von Hybridsorten ist die gezielte Bestäubung einer Mutterlinie mit einer ausgewählten Vaterlinie als Pollenspender. Um ausreichende Mengen an Saatgut zu erzeugen, muss dieser letzte Schritt im großen Maßstab unter Freilandbedingungen erfolgen. Durch Anbau einer pollenlosen, d.h. männlich sterilen Mutterlinie und einer in direkter räumlicher Nähe angebauten fertilen (pollentragenden) Vaterlinie wird eine derartige gelenkte Bestäubung herbeigeführt.

Dabei entstehen in großem Umfang Hybridsamen, die ausschließlich auf die Kreuzungskombination der beiden Elternlinien zurückgehen. Voraussetzung hierfür ist insbesondere das Vorhandensein einer vollständig, d.h. möglichst 100%ig männlich sterilen Mutterlinie, die sich nicht selbst bestäuben kann.

Aus diesem Grunde wurden in der Vergangenheit verschiedene Methoden, insbesondere mechanische, chemische und genetische Verfahren zur Induktion von männlicher Sterilität von Pflanzen entwickelt. Mechanische Methoden, wie beispielsweise das Entfernen der Antheren, kommen nur bei Pflanzenarten mit großen und/oder räumlich getrennten Geschlechtsorganen, wie z.B. dem Mais (*Zea mays* L.), in Frage. Zur chemischen Emaskulation von Pflanzen wurden als Gametozide bezeichnete Substanzen entwickelt, die nach Applikation pollenabtötend wirken. Auf diesem Wege konnte auch bei strengen Selbstbefruchtern wie Weizen und Gerste erstmals Hybridsorten erzeugt werden.

Genetische Mechanismen, die männliche Sterilität von Pflanzen induzieren, sind bereits seit langem beschrieben. So treten grundsätzlich männlich sterile Pflanzen in den zumeist aneuploiden Nachkommenschaften von weiten, d.h. interspezifischen oder intergenerischen Kreuzungen auf. Dies ist z.T. auf Unregelmäßigkeiten in der Meiose der Nachkommenschaft zurückzuführen und betrifft die männlichen und weiblichen Gameten gleichermaßen. Daneben wurden aber auch Systeme entdeckt und weiterentwickelt, die auf einzelnen Gendefekten beruhen und ausschließlich die männlichen Gameten bzw. den Pollen beeinflussen. Solche Systeme gehen einerseits auf Mutationen im Kemgenom (nuclear male sterility, NMS) und andererseits auf Genveränderungen im Plastom bzw. Cytoplasma (cytoplasmatic male sterility, CMS) zurück.

Die CMS beruht im Prinzip auf einer Inkompatibilität zwischen Zellkern und Cytoplasma und wird bei den meisten höheren Pflanzen strikt maternal vererbt (U. Witt, Identifikation und Charakterisierung eines kernkodierten Mitochondrienproteins aus dem pollensterilität-induzierenden Polima-Cytoplasma von Brassica napus L., Dissertation, Hamburg 1993.).

Unter Zuhilfenahme einer entsprechenden Vaterlinie, die die Sterilität des mütterlichen Cytoplasmas nicht aufzuheben vermag (sog. 'Maintainer'), kann theoretisch nach mehrmaliger Rückkreuzung mit der Maintainerpflanze eine reinerbige, sterile CMS-Pflanze erzeugt werden. Ein vollständiges CMS-System beispielsweise zur Erzeugung von Hybridsaatgut bei Gräsern, deren vegetative Masse genutzt wird, besteht demzufolge aus folgenden Komponenten:
1. der CMS-Linie, die ein sterilitätsinduzierendes Cytoplasma (S) trägt, auch sterile Mutterlinie genannt.
2. der Maintainer-Linie, die normal fertiles Cytoplasma (N) trägt und der CMS-Linie ansonsten sehr ähnlich ist.
3. der Bestäuber- oder Vaterlinie, die normal fertil ist und eine gute Kombinationseignung zur CMS-Mutterlinie besitzt.

Ein grundlegendes technisches Problem bei der Erzeugung von Hybridsorten stellt die Stabilität der männlichen Sterilität der CMS-Linie dar. Dies betrifft insbesondere die 100%ige Weitergabe der männlichen Sterilität in die nächste Generation nach erfolgter Kreuzung und die Bereitstellung eines umweltunabhängigen Phänotyps in Form von männlich sterilen Pflanzen. Nur unter diesen Bedingungen können agronomisch optimierte und vollständig männlich sterile Mutterpflanzen erzeugt werden, die es ermöglichen, Heterosis in Form von Hybridsorten in vollem Umfang zu nutzen und ein zusätzliches Ertragspotential zu realisieren.

Die Lolium-Arten Deutsches Weidelgras (*Lolium perenne* L.), Welsches Weidelgras (*Lolium multiflorum* L.) und Bastardweidelgras (*Lolium hybridum* L.) sind die bedeutendsten Grasarten im europäischen Futtergrasanbau. Bei Futtergräsern wird in der zielgerichteten Ausnutzung von Heterosiseffekten eine reale Möglichkeit zu einer wesentlichen Erhöhung des Ertragspotentials und zur Verbesserung weiterer quantitativer Eigenschaften wie Stresstoleranzen gegen biotische und abiotische Faktoren gesehen. Da die vorstehend genannten Lolium-Arten Fremdbefruchter sind, bietet sich zu diesem Zweck die Züchtung von Synthetics und Hybridsorten an.

Um zusätzlich Variabilität als Ausgangsbasis zur Selektion neuer Genotypen zu erlangen, wird in der Züchtung von Kulturpflanzen das Verfahren der Polyploidisierung genutzt.. Bei der Polyploidisierung wird durch den Einsatz von Mitosehemmern wie Colchicin während der Mitose eine Aufdoppelung des Chromosomensatzes einer Zelle bewirkt. Dies hat bei Lolium-Arten zur Folge, dass aus den ursprünglich diploiden Arten (2n = 2x =14) tetraploide Formen erzeugt werden, die einen doppelten Chromosomensatz besitzen (2n = 4x = 28). Da Tetraploide andere Eigenschaften als Diploide aufweisen, wurden für die wirtschaftlich relevanten *Lolium-Arten L. perenne, L. multiflorum* und *L. hybridum* entsprechende tetraploide Sorten gezüchtet.

Für die Weidelgrasarten gibt es eine Reihe von Untersuchungen, die auf Heterosis und Hybridwüchsigkeit in allen Ploidiestufen hinweisen (u.a. C. A. Foster, Interpopulational and intervarietal hybridization in Lolium perenne breeding, heterosis under noncompetitive conditions, J. Agric. Sci. 1971, 107-130; C. A. Foster, (1973): Interpopulational and intervarietal F1-Hybrids in Lolium perenne: performance in field sward conditons, J. Agric. Sci 1973, 80, 463-477; I. Rod, Beitrag zu den methodischen Fragen der Heterosiszüchtung bei Futtergräsern, Ber. Arbeitstagung Arbeitsgemeinsch. Saatzuchtleiter Gumpenstein 1965, S. 235-252; I. Rod, Remarks on heterosis with grasses, Heterosis in plant breeding, Proc. 7 th Congr. Eucarpia Budapest (1967), S. 227-235; A. J. Wright, A theoretical apprasial of relative merits of 50% hybrid and synthetic, J. Agric. Sci 79, 1972, S. 245-247). Insbesondere konnten in der Vergangenheit nach Einzelpflanzen-, Linien- und Sortenkreuzungen Heterosiseffekte festgestellt werden (Kobabe, siehe oben).

Die gegenwärtig am häufigsten angewandte Zuchtmethode, nämlich die Erzeugung von Synthetics oder Sorten auf Klon- oder Populationsbasis, wurde 1940 von Frandsen (N. H. Frandsen, Some breeding experiments with timothy, Imp. Agric. Bur. Joint Publ. 1940, 3, 80-92) bei Gräsern entwickelt. Dieses Verfahren ermöglicht allerdings wie vorstehend erwähnt nur eine teilweise Nutzung der Heterosis. Eine echte Futtergras-Hybridsorte durch Verwendung einer *Lolium-Linie* mit cytoplasmatischer männlicher Sterilität zur vollständigen Nutzung der Heterosis (C. Bothe, siehe oben; G. Kobabe, siehe oben; V. Lein, siehe oben) ist bisher nicht bekannt, da keine Pflanzen mit vollständiger männlicher Sterilität zur Verfügung standen und die bekannten CMS-Quellen instabil sind.

Die bei den Lolium-Arten gefundenen bzw. angewendeten Systeme zur Erlangung der männlichen Sterilität unterscheiden sich hinsichtlich ihrer Entstehung und Wirkungsweise. Systeme mit mechanischer Kontrolle zur Kastration der Pflanzen scheiden bei den *Lolium-Arten* morphologiebedingt aus. Chemische Methoden wurden bislang bei *Lolium* nicht entwickelt, während genetische Kontrollmechanismen bereits vor geraumer Zeit beschrieben wurden. Über spontan entstandene Quellen wird bei *Lolium multiflorum* von Nitzsche (Cytoplasmatische männliche Sterilität bei Weidelgras (Lolium ssp.) Z. Pflanzenzücht., Berlin (West) 65, (1971), S. 206-220) und bei *Lolium perenne* von Gaue (Möglichkeiten der Hybridzüchtung auf ms-Basis bei *Lolium perenne* L. XIII. Internat. Grasland-Kongreß, Leipzig 1977, Sektionsvortrag 1-2, S. 491-496; Ergebnisse von Untersuchungen zur Hybridzüchtung bei (Lolium perenne Tag.-Ber., Akad. Landwirtsch.-Wiss. DDR, Berlin (1981) 191, S. 119-126) berichtet. Nach Art- und Gattungskreuzungen entstanden ebenfalls bei *Lolium perenne* männlich sterile Formen (F. Wit, Cytoplasmic male sterility in ryegrasses (Lolium ssp.) detected alter intergeneric hybridization, Euphytica 1974, 23, 31-38; V. Connoly, Hybrid grasses varieties for the future Farm Food Res. 1978, 9, 6, 131-132; V. Connoly, R. Wright-Turner, Induction of cytoplasmic male-sterility into ryegrass (Lolium perenne), Theor. Appl. Genet. 1984, 68, 449-453). Keines dieser genetischen Systeme konnte allerdings geno- und phänotypisch stabilisiert werden, so dass bislang kein funktionsfähiges Hybridsystem in den verschiedenen Weidelgrasarten bekannt ist.

Obwohl an der Erzeugung von Hybridlinien mit verbesserten agronomischen Eigenschaften intensiv geforscht wird, führen die bisher zur Erzeugung von männlich sterilen Pflanzen zur Verfugung stehenden Verfahren jedoch in vielen Fällen nicht zu voll befriedigenden Ergebnissen. Es besteht somit ein starkes Bedürfnis nach einem Verfahren zur Erzeugung von vollständig männlich sterilen und stabilen Pflanzen, welche die Nachteile des Standes der Technik nicht aufweisen.

Der vorliegenden Erfindung liegt somit die Aufgabe zugrunde, Verfahren zur Herstellung von stabilen, d.h. u.a. umweltunabhängigen und vollständig, d.h. zu 100% männlich sterile Pflanzen der Gattung *Lolium* zur Verfügung zu stellen, die es ermöglichen, gezielt Hybridpflanzen zu erzeugen und damit Heterosiseffekte wie beispielsweise ein zusätzliches Ertragspotential auszunutzen.

Diese und weitere Aufgaben der Erfindung werden durch die Bereitstellung der in den Patentansprüchen gekennzeichneten Ausflümmgsformen gelöst.

Erfindungsgemäß wird ein Verfahren bereitgestellt, mit dem es jetzt erstmals möglich ist, vollständig, d.h. zu 100% männlich sterile Pflanzen der Gattung *Lolium* (im folgenden auch als MSL oder MSL-Pflanze (männliche Sterilität *Lolium* bezeichnet) herzustellen. Diese völlig neuartige Pflanze der Gattung *Lolium* zeichnet sich durch eine hohe Stabilität des sterilitätsverursachenden Plasmas aus. Insbesondere ist die bei den durch das erfindungsgemäße Verfahren hergestellten Pflanzen erzeugte männliche Sterilität temperaturstabil und weist nur eine äußerst geringe Umweltabhängigkeit auf. Im Gegensatz zu bisher bekannten sterilen Kreuzungsnachkommen von Pflanzen mit männlicher Sterilität, bei denen ein hoher Anteil an teilsterilen Pflanzen in Abhängigkeit von der Antherenausprägung vorliegt, ist der Sterilitätsgrad bei MSL unabhängig von der Antherenausprägung gleichmäßig hoch (Tabellen 2 bis 4). Die durch das erfindungsgemäße Verfahren erzeugten MSL-Linien ermöglichen folglich erstmals die Produktion von homogenen *Lolium*-F₁-Hybridsorten unter Freilandbedingungen.

Das erfindungsgemäße Verfahren zur Herstellung von vollständig männlich sterilen Pflanzen der Gattung *Lolium* umfasst die folgenden Schritte:
a) Mutagenese von Samenmaterial von Wildtyppflanzen der Gattung *Lolium*;
b) Untersuchung der mutagenisierten *Lolium-Pflanzen* mit Southern-Blot-Teckniken; und
c) Identifizierung von vollständig männlich sterilen *Lolium-Pflanzen*,
   dadurch gekennzeichnet, dass die Primerpaare zur Amplifizierung der bei der Southern-Blot-Hybridisierung eingesetzten Sonden aus der Gruppe, bestehend aus den folgenden Primerpaaren ausgewählt werden:
   a) TTACTTCACATAGCTTTTCGTU
      CCACAAACCACAAGGATATAG
   b) ATGATTGAATCTCAGAGGCAT
      CATATACCTCCCCACCAATAG
   c) TTAGTAGATCGTGAGTGGGTC
      GTGCTAAAAATCCGGTACAT
   d) TTATCCGTCGCTACGCTGTTC
      AATGGAAAGATCGGAACATGG
   e) ATGACTATAAGGAACCAACGA
      GATCAGTCTCATCCGTGTAA
   f) ATGAGACGACTTTTTCTTGAA
      CTTGTAAACTAATCGAGACCG,
   wobei die Sequenzen in 5'-3'-Richtung angegeben sind und jeweils die erste Sequenz den oberen Primer darstellt,
   und die durch die Amplifikation mit den Primern hergestellten Sonden in einer der folgenden Kombinationen mit Restriktionsenzymen zur Southern-Blot-Analyse eingesetzt werden:
   a) zusammen mit HindIII oder DraI
   b) zusammen mit HindIII, DraI oder EcoRV
   c) zusammen mit HindIII oder BamHI
   d) zusammen mit HindIII, XbaI, DraI, EcoRV, BamHI oder HaeIII
   e) zusammen mit XbaI oder HaeIII
   f) zusammen mit EcoRV.

Unter Wildtyppflanzen werden im Rahmen der vorliegenden Erfindung natürlich vorkommende Pflanzen der Gattung *Lolium* verstanden, insbesondere solche, deren Erbmaterial nicht durch Mutagenese manipuliert wurde. Bei dem Samenmaterial handelt es sich insbesondere um Karyopsenmaterial.

Die Mutagenese in Schritt a) des erfindungsgemäßen Verfahrens erfolgt vorzugsweise durch Behandlung des Samenmaterials mit chemischen Mutagenen. Besonders bevorzugt wird N-Ethylharnstoff zu diesem Zweck eingesetzt. Weitere denkbare Mutagene sind alkylierende Agenzien wie Methylmethansulfonat und Diepoxybutan, Urethane, Nitrosoverbindungen, Alkaloide wie Colchicin, Peroxide wie H₂O₂, Alkylperoxide und dergleichen.

Bei einer alternativen Ausführungsform erfolgt die Mutagenese durch Bestrahlung des Samenmaterials mit kurzwelliger UV-Strahlung (z.B. 254 nm); langwelliger UV-Strahlung (z.B. 300 bis 400 nm) in Kombination mit Psoralenen; ionisierender Strahlung wie Röntgen- und γ-Strahlung; und dergleichen.

Die *Lolium-Pflanzen,* in denen die cytoplasmatische männliche Sterilität erzeugt wird, werden vorzugsweise ausgewählt aus der Gruppe, bestehend aus *Lolium perenne, Lolium multiflorum* und *Lolium hybridum.*

Die Untersuchung der mutagenisierten *Lolium-Pflanzen* mit auf die Pollenvitalität gerichteten Testmethoden dient der Differenzierung von fertilem und dem gewünschten sterilen Pollen. Aufgrund der guten Übereinstimmung zwischen Antherenausprägung und Sterilitätsgrad - 99% der durch das erfindungsgemäße Verfahren erzeugten Pflanzen, die visuell als steril eingestuft worden sind, sind tatsächlich vollsteril - sind Pollenvitalitätsuntersuchungen bei dem erfindungsgemäßen Verfahren lediglich erforderlich, falls eine Kontrolle gewünscht ist. Bei den aus dem Stand der Technik bekannten Pflanzen mit männlicher Sterilität ist dagegen keine sichere visuelle Bewertung der erzeugten männlichen Sterilität möglich, da dort keine bzw. nur eine geringe Übereinstimmung zwischen Antherenausprägung und Sterilitätsgrad vorliegt (Tabelle 3).

Vorzugsweise sind die Testmethoden Färbemethoden, wie beispielsweise die Methode nach Alexander (M.P. Alexander, Differential staining of aborted and nonaborted pollen, Stain Technology 1969, 44/3, 117-122), die Zugabe von Lichtgrünreagenz (I. Sinska, Ergebnisse der Forschung der Pollensterilität der Luzerne d'Eucarpia-Groupe Medicago sativa Piestany 17.-21.5.1976) und die Zugabe von Lugolscher Lösung. Die entsprechenden Reagenzien für die vorstehend genannten Färbemethoden sind in Tabelle 1 aufgeführt.

Primerpaare zur Amplifizierung der bei der Southern-Blot-Hybridisierung eingesetzten Sonden werden vorzugsweise ausgewählt aus der Gruppe, bestehend aus den folgenden Primerpaaren (siehe auch Tabelle 6):
a) TTACTTCACATAGCTTTTCGTU und
   CCACAAACCACAAGGATATAG;
b) ATGATTGAATCTCAGAGGCAT und
   CATATACCTCCCCACCAATAG;
c) TTAGTAGATCGTGAGTGGGTC und
   GTGCTAAAAATCCGGTACAT;
d) TTATCCGTCGCTACGCTGTTC und
   AATGGAAAGATCGGAACATGG;
e) ATGACTATAAGGAACCAACGA und
   GATCAGTCTCATCCGTGTAA;
f) ATGAGACGACTTTTTCTTGAA und
   CTTGTAAACTAATCGAGACCG;
wobei die Sequenzen in 5'-3'-Richtung angegeben sind und jeweils die erste Sequenz den oberen Primer darstellt.

Für die Southern-Blot-Hybridisierung bei dem erfindungsgemäßen Verfahren bevorzugte Restriktionsenzyme werden ausgewählt aus der Gruppe, bestehend aus *Hind*III, *Xb*aI, *Dra*I, *Eco*RV, *Bam*HI und *Hae*III.

Mit Hilfe von Kombinationen der vorstehend beschriebenen Sonden und Enzyme, insbesondere durch Kombination der Sonde *nad9* (siehe Tabelle 6) mit dem Restriktionsenzym *Dra*I ist es möglich, Pflanzen mit stabiler und instabiler männlicher Sterilität bzw. vollständiger und nicht vollständiger männlicher Sterilität eindeutig zu unterscheiden.

In natürlichen *Lolium-*Populationen liegt ein differenzierter Anteil an Maintainer-Pflanzen vor, der durch entsprechende Testkreuzungen mit Überprüfung der F₁-Generation auf Sterilität ermittelt wird.

Eine stabile MSL-Linie wird vorzugsweise durch mehrfache Rückkreuzung mit Maintainer-Linien erhalten.

Das sterilitätsinduzierende Plasma der diploiden MSL-Linie wird bei einer weiteren bevorzugten Ausführungsform der vorliegenden Erfindung durch Polyploidisierung in die tetraploideGenomstufe gebracht. Die Merkmalsausprägung auf tetraploider Stufe ist analog der diploiden Stufe.

Die Polyploidisierung erfolgt durch Behandlung der MSL-Pflanzen mit Mitosehemmem wie beispielsweise Chalonen, Colchicin, Narcotin, Chinonen und dergleichen. Besonders bevorzugt erfolgt die Polyploidisierung durch Behandlung mit Colchicin.

Beispielsweise liegen in *L. perenne* und *L. multiflorum* sowohl tetraploide als auch diploide Formen vor. Da die Art *L. hybridum* eine direkte Kreuzung der beiden vorstehend genannten Arten darstellt, ist das MSL-System auch in dieser Art in beiden Genomstufen nutzbar.

Des Weiteren ist durch Kreuzungen eine Übertragung des *Lolium perenne-MSL-*Plasmas auf *Lolium-Arten* wie *Lolium hybridum* und *Lolium multiflorum* möglich. Somit wird durch die vorliegende Erfindung die Möglichkeit bereitgestellt, bei genannten *Lolium-Arten* Hybridpflanzen auf der Basis einer *Lolium*-Linie mit stabiler und vollständiger männlicher Sterilität zu erzeugen.

Sowohl Maintainer, MSL-Linie und Bestäuber (= fertile Vaterlinie, die zur Erzeugung der F1-Hybride genutzt wird) sind vorzugsweise bei *L. perenne* und *L*. *multiflorum* von der selben Art. Da *L. hybridum* per se eine Kombination der beiden erstgenannten Arten darstellt, können alle drei Komponenten (Maintainer, MSL-Linie und Bestäuber) jeweils aus den beiden Grundarten kombiniert werden. Maintainer können nach herkömmlichen Techniken aus käuflichen Sorten und Zuchtstämmen isoliert bzw. entwickelt werden. Der Bestäuber kann beliebig gewählt werden, da die Fertilität der eigentlichen F1-Hybride keine Rolle spielt, weil bei Futtergräsem nur die vegetative Masse genutzt wird.

Durch die erfindungsgemäßen Verfahren konnten somit MSL-Linien hergestellt werden, die im Vergleich zu bisher bekannten männlich sterilen *Lolium-Pflanzen* einen hohen Grad an männlicher Sterilität und Stabilität aufweisen und sich somit substantiell von diesen unterscheiden. Mit Hilfe der erfindungsgemäßen MSL-Linien ist es damit erstmals möglich, F₁-Hybridsorten von Lolium-Arten, insbesondere der Futtergrasarten *Lolium perenne*, *Lolium multiflorum* und *Lolium hybridum* zu erzeugen.

Des Weiteren wird durch die vorliegende Erfindung erstmals ein Verfahren bereitgestellt, mit dem MSL-Pflanzen durch Southern Blot-Hybridisierung anhand des Hybridiserungsmusters von *Lolium-* Pflanzen mit teilweiser oder instabiler männlicher Sterilität unterschieden werden können.

Die vorliegende Erfindung wird in den nachfolgenden Beispielen veranschaulicht, ohne sie in irgendeiner Weise einzuschränken.

### Beispiele

### Beispiel 1:

### Mutagenisierung des Karyopsenmaterials von Lolium-Pflanzen

Das Ausgangsmaterial für die experimentelle Mutagenese war ein diploider *L. perenne* Wildtyp. Die Karyopsen wurden unter Nutzung des mutagenen Agens N-Ethylharnstoff (C₃H₈N₂O) in einer Konzentration von 0,025% für 18 Stunden inkubiert.

Aus den so behandelten 20.000 *L. perenne*-Einzelsamen konnten 1200 potentiell mutierte Einzelpflanzen (erste Generation nach Mutagenese = M₁) angezogen werden. Die restlichen Karyopsen keimten entweder nicht aus oder die Keimlinge waren nicht lebensfähig.

Es folgte eine visuelle Bonitur der 1200 M₁-Pflanzen auf Pollenschüttung, wobei zunächst 20 Einzelpflanzen als steril eingeordnet wurden. Die so vorselektierten Einzelpflanzen wurden dann drei verschiedenen, auf die Pollenvitalität gerichteten Testverfahren unterzogen (siehe Tabelle 1): 1. Methode nach Alexander (siehe oben), 2. Lichtgranreagenz ( 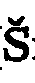 inska, siehe oben), 3. Lugolsche Lösung. Nach erfolgter Untersuchung wurden 19 Einzelpflanzen als teilsteril und eine Einzelpflanze (M 361) als vollsteril eingestuft.

Basierend auf der vollsterilen Mutante M 361 wurde eine stabile MSL-Linie in diploidem Deutschem Weidelgras durch Rückkreuzung mit Maintainer-Linien entwickelt, die im Folgenden mit MSL-19 bezeichnet ist.

Nach erfolgter Polyploidisierung konnte die cytoplasmatische männlichen Sterilität auch in der tetraploiden Stufe etabliert werden. Die tetraploide MSL-Linie ist im Folgenden mit MSL-163 bezeichnet.

Durch mehrfache Rückkreuzung gelang eine Übertragung des MSL-Plasmas von *L. perenne auf L. multiflorum.* Auch hier wurde MSL-Material in diploider und tetraploider Stufe erzeugt.

### Beispiel 2:

### Pollenbonitur der Pflanzen

Die Charakterisierung der männlichen Sterilität der erfindungsgemäßen MSL-Linie mit vollständiger cytoplasmatischer männlicher Sterilität erfolgte anhand einer Antheren- und Pollenbonitur und im Vergleich mit bereits bekannten CMS (cytoplasmatic male sterility)-Pflanzen aus *Lolium perenne.* Als direkter Vergleich diente zunächst die *L. perenne* CMS-Linie CMS-1 (D. Burkert, R. Schlenker, Pollensterilität bei Lolium perenne L. und Festuca pratensis Hunds. Wiss. Z. Univ. Rostock math.-naturwiss. R., 1975, 4. 7, 845-850; I. Gaue, Ergebnisse von Untersuchungen zur Hybridzüchtung bei Lolium perenne Tag.-Ber., Akad. Landwirtsch.-Wiss. DDR, Berlin (1981) 191, 119-126).

Überraschenderweise war der Sterilitätsgrad der durch das erfindungsgemäße Verfahren hergestellten MSL-Linie unabhängig von der Antherenausprägung der einzelnen MSL-Pflanzen gleichmäßig hoch, wobei zudem eine sichere Vererbung des sterilitätsinduzierenden Plasmas bei MSL gefunden wurde (siehe Tabellen 2 bis 4). Das unterscheidet sie von den aus dem Stand der Technik bekannten sterilen Kreuzungsnachkommen der CMS-Quellen.

MSL-19 zeichnete sich durch eine reduzierte Antherengröße ohne lebensfähigen Pollen aus. Letzteres wurde durch die genannten Färbeverfahren ebenfalls nachgewiesen. Die Merkmalsausprägung von MSL auf tetraploider Stufe (MSL-163) war analog der diploiden Stufe.

Neben der CMS-Quelle CMS-1 wurden die Sorte Inca (CMS-I1 bis -14), welche auf einem CMS-System beruht, sowie Pflanzen von sieben CMS-Herkünften der Landessaatzuchtanstalt Hohenheim (CMS-112, CMS-113, CMS-114, CMS-115, CMS-117, CMS-118, CMS-119) in die fortführenden Untersuchungen einbezogen, um einen Vergleich mit weiteren zur Verfügung stehenden *Lolium*-CMS-Pflanzen zu ermöglichen. Dazu wurde unter zwei verschiedenen Umweltbedingungen, nämlich Gewächshaus und Freiland, Pollen reifer Antheren der Einzelpflanzen CMS-1 (S), CMS-1 (N) sowie, stellvertretend für MSL, die Einzelpflanzen der Linie MSL-163 (S) untersucht und miteinander verglichen. Des Weiteren wurde der Pollen der Einzelpflanzen CMS-112 bis -115 sowie CMS-117 bis -119 bonitiert.

### a) CMS-1(S)

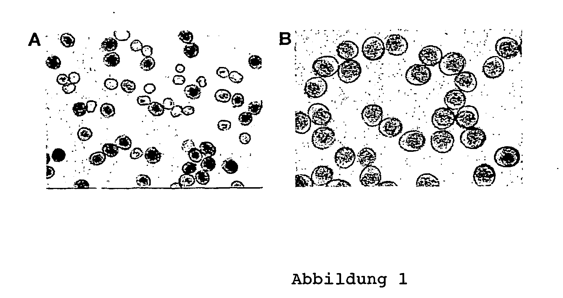

Insgesamt wurden 40 Einzelpflanzen CMS-1 (S) untersucht. Dabei wurde gezeigt, dass neben vollkommen sterilen Pflanzen auch semisterile mit Anteilen von fertilem Pollen zwischen 20% und 75% (Abbildung 1A) als auch vollständig fertile Pflanzen (Abbildung 1B) auftraten.

Das unterschiedliche Verhältnis von fertilem und sterilem Pollen bei semisterilen Pflanzen ist höchstwahrscheinlich durch umweltbedingte Faktoren hervorgerufen worden, da Einzelpflanzen unter unterschiedlichen Umweltbedingungen einen unterschiedlichen Anteil an fertilem Pollen produzierten. Letzteres bestätigt die Instabilität der Quelle CMS-1.

### b) CMS-1 (N)

Von 34 untersuchten Einzelpflanzen CMS-1 (N) wurden 32 als vollständig fertil bonitiert, 2 Einzelpflanzen dagegen als semisteril, d.h. im Quetschpräparat konnten sowohl fertiler als auch steriler Pollen beobachtet werden.

### c) MSL-163 (S)

Innerhalb MSL-163 (S) wurden 20 Einzelpflanzen untersucht und alle als vollständig steril bonitiert. Die Stabilität der CMS in dieser Linie könnte auf einem frühen Abbruch der Pollenentwicklung beruhen; anfärbbare Pollenkörner wurden in keinem Fall beobachtet.

### d) CMS-112 bis -115, CMS-117 bis CMS-119

Die Pollenbonitur von Einzelpflanzen jeder Herkunft ergab, dass der Pollen der Herkünfte CMS-112, CMS-113, CMS-114, CMS-115, CMS-117, CMS-118 und CMS-119 als semisteril einzustufen ist, d.h. bei allen Pflanzen liegen neben sterilen auch anfärbbare, also fertile Pollenkörner vor. Dies lässt sich, ähnlich wie bei CMS-1 (S), durch eine Instabilität der cytoplasmatischen männlichen Sterilität erklären.

### Beispiel 3:

### Molekularbiologische Untersuchungen von MSL-Pflanzen und Vergleichspflanzen mit cytoplasmatischer männlicher Sterilität

Ergänzend zu den phänotypischen Daten (Pollenbonitur aus Beispiel 2) wurden zur sicheren genotypischen Unterscheidung von MSL gegenüber bekannten CMS-Systemen in *Lolium* das Verfahren einer Southern-Hybridisierung angewendet.

### a) Isolierung von Gesamt-DNA

Die Isolierung der Gesamt-DNA erfolgte im Wesentlichen nach Wilkie (Isolation of total genomic DNA. In: M. S. Clark (Hrsg.) Plant Molecular Biology - A Laboratory Manual. 1997, Springer Verlag, Berlin Heidelberg New York, 3-14). In einem mit flüssigem Stickstoff (LN₂) vorgekühlten Edelstahlzylinder wurden 1 bis 2 g LN₂-gefrorenes Blattmaterial mit einer Kugelschwingmühle (Fa. Retsch, Haan) zu einem feinen Pulver zermahlen und in 50 ml-Reaktionsgefäße überfährt. Danach folgte eine Inkubation in 20 ml 2x CTAB-Puffer im Wasserbad bei 65°C für 90 min und anschließende zweimalige Extraktion mit Chloroform/Isoamylalkohol (24:1, v/v). Nach Zentrifugation bei 5000 x g für 15 min in einem Festwinkelrotor HFA 13.50 (Fa. Heraeus, Hanau) wurde die wässrige Phase in neue 50 ml-Reaktionsgefäße überführt. Der RNA-Abbau erfolgte durch Zugabe von 1/100 Vol. RNase-Lösung (10 mg/ml) und Inkubation für 30 min bei 37°C. Die DNA-Präzipitation erfolgte durch Zugabe von 0,7 Vol-%. Isopropanol und Inkubation bei Raumtemperatur für 20 min. Mit Hilfe einer Pasteurpipette wurde die DNA in 10 ml 76% Ethanol/0,2 M NaOAc überführt und für 20 min auf Eis inkubiert. Danach erfolgte ein weiterer Waschschritt für 5 min in 2 ml 70% Ethanol auf Eis. Anschließend wurde die DNA durch Zentrifugation bei 13000 x g für 4 min (Biofuge 22 R, Fa. Heraeus) pelletiert. Nach Aspiration des überschüssigen EtOH wurde das Pellet bei RT getrocknet und in TE-Puffer gelöst (Volumen ist abhängig von der Pelletgröße). Die Bestimmung der DNA-Konzentration erfolgte photometrisch bei 260 nm (GeneQuant II, Fa. Pharmacia Biotech, Freiburg). Alle zur DNA-Isolierung verwendeten Puffer sind in Anhang A aufgeführt.

### b) Isolierung von mitochondrialer DNA

Die Isolierung von mtDNA erfolgte in Anlehnung an die Protokolle von Kiang et al. (Cytoplasmic male sterility (CMS) in Lolium perenne L.: 1. Development of a diagnostic probe for the male-sterile cytoplasm. Theor Appl Genet 1993, 86, 781-787) und Chase und Pring (Properties of the linear N1 and N2 plasmid-like DNAs from mitochondria of cytoplasmic male-sterile Sorghum bicolor. Plant Mol Biol 1986, 6, 53-64). Als Ausgangsmaterial wurde frische Blattmasse von Gewächshauspflanzen verwendet, die zuvor für 16-20 h mit schwarzer Folie abgedunkelt worden waren. Alle Arbeitsschritte bis zur Mitochondrienlysis wurden bei 4°C durchgeführt. Im ersten Arbeitsschritt wurden 60 g Blattmasse in 400 ml Extraktionspuffer in einem Standmixer (Gastronom GT95, Fa. W. Krannich, Göttingen) zerkleinert, durch 5 Lagen Verbandmull (YPSIGAZE-8-fach, Fa. Holthaus-Medical, Remscheid) gefiltert und anschließend für 10 min bei 5000 x g zentrifugiert. Der Überstand wurde in neue Zentrifugenröhrchen dekantiert und zur Mitochondrienpelletierung erneut für 10 min bei 16000 x g zentrifugiert. Das Mitochondrienpellet wurde anschließend mit Hilfe eines sterilen Haarpinsels in 8 ml DNAse-Puffer resuspendiert. Nach Zugabe von 8 mg DNAse I erfolgte der Abbau der Kern- und Plastiden-DNA (90 min bei 4°C). Im nächsten Schritt wurde die Mitochondriensuspension mit 20 ml Waschpuffer unterschichtet und bei 12000 x g (30 min) zentrifugiert. Es erfolgte eine weitere Resuspension des Pellets in Waschpuffer mit anschließender Zentrifugation.

Die Mitochondrien wurden in 3 ml Lysispuffer unter Zugabe von Proteinase K (100 µg/ml Endkonzentration) und 0.5% SDS bei 37°C (lh) lysiert. Anschließend wurden 3 ml Extraktionspuffer dazugegeben und die Proben bei 65 °C im Wasserbad für 10 min inkubiert.
Nach Aliquotierung der Proben (600 µl) in Eppendorf-Reaktionsgefäße (2 ml) erfolgte die Proteinfällung durch Zugabe von 200 µl 5 M Kaliumacetat auf Eis (20 min). Die Proteine wurden durch Zentrifugation der Proben für 5 min bei 13000 U/min pelletiert, die Überstände in neue Reaktionsgefäße überfuhrt und die mtDNA durch Zusatz von 400 µl Isopropanol und 40 µl 5 M Ammoniumacetat über Nacht bei -20°C präzipitiert. Nach 5 min Zentrifugation mit 13000 U/min wurde die mtDNA pelletiert und in 100 µl "50-10"TE-Puffer resuspendiert.

Danach erfolgte die Zusammenführung mehrerer Aliquots zu einem Volumen von 500 µl und ein RNA-Abbau durch Zugabe von RNase A (100 µg/ml Endkonzentration) für 1 h bei 37°C. Die emeute Präzipitation der mtDNA erfolgte wie oben beschrieben, das DNA-Pellet wurde in 100 µl TE-Puffer gelöst. Die Konzentrationsbestimmung wurde wie bei der Gesamt-DNA durchgeführt. Alle zur mtDNA-Isolierung verwendeten Puffer sind in Anhang A aufgeführt.

### c) Restriktion, Elektrophorese und Blotting

Für die Hybridisierungsversuche wurden 5 µg Gesamt- oder mtDNA mit 5 U Restriktionsenzym unter Zugabe der entsprechenden Reaktionspuffer für mindestens 4 h bei 37°C restringiert. Als Restriktionsenzyme wurden die Endonukleasen *Hind*III, *Bam*HI, *Eco*RV, *Xba*I, *Dra*I und *Hae*III (Fa. Gibco BRL, Eggenstein) verwendet.

Die DNA-Fragmente wurden nach Zugabe von 5x Ladepuffer in einem 1%-Agarosegel für 6-8 h bei 50-60 V in 1x-TAE-Puffer aufgetrennt und anschließend in einer Ethidiumbromidlösung (0,1 mg/ml) angefärbt. Die Fragmentgrößenbestimmung erfolgte mit Hilfe eines DIG-markierten DNA-Längenstandards (Fa. Roche, Granzach-Wyhlen).

Anschließend wurde die DNA auf eine positiv geladene Nylonmembran (Fa. Roche) durch Kapillarblot übertragen. Der Transfer erfolgte über Nacht, als Transfermedium diente eine 20 x SSC-Lösung.

Nach dem DNA-Transfer wurden die Filter in 2x-SSC (10 min) gewaschen und bis zur Hybridisierung feucht in Plastikfolie eingeschweißt. Zur Fixierung der DNA erfolgte eine UV-Bestrahlung (30 s; 0,120 J/cm²).

### d) Beschreibung der Sonden

Die Primer zur Amplifikation der benötigten Gensonden wurden aus cDNA-Sequenzen des mitochondrialen Genoms von *Arabidopsis thaliana* nach Datenbankrecherche (EMBL; ID Miatgen) abgeleitet. Dabei wurden unterschiedliche Gene, welche für ribosomale Proteine und Untereinheiten der Proteinkomplexe der Atmungskette kodieren, ausgewählt (siehe Tabelle 5).

Zur Kontrolle auf Kontamination isolierter mtDNA mit genomischer DNA wurde eine ubiquitäre kemgenomische cDNA-Gensonde (Actin) verwendet. Die Primer wurden von bekannten Actin-cDNA-Sequenzen aus Reis abgeleitet; die Amplifikation erfolgte aus Roggenpollen-cDNA.

### e) Herstellung der Sonden

Die Primerpaare zur Amplifizierung der eingesetzten Sonden wurden aus cDNA-Sequenzen der entsprechenden Gene mit Hilfe des Computerprogramms OLIGO 5.0 abgeleitet (siehe Tabelle 6). Die Markierung der Sonden erfolgte durch den Einbau von Digoxigenin-dUTP (Fa. Roche) während der PCR-Reaktion mit einem Thermocycler, Modell UNO, der Fa. Biometra (Göttingen).

Die Reaktionsparameter sind in den Tabellen 7 und 8 angegeben.

### f) Nichtradioaktive Southern-Hybridisierung

Für die nichtradioaktive Southern-Hybridisierung wurde das DIG-System der Fa. ROCHE verwendet. Die Reaktion erfolgte in Hybridisierungsröhren in einem Hybridisierungsofen (Fa. Stuart Scientific, Staffordshire, UK). Die Prähybridisierung von 1-2 Filtern je Röhrchen wurde in 20 ml DIG-Easy-Hyb (Fa. Roche) für mindestens 1 h durchgeführt. Anschließend wurde die Prähybridisierungslösung gegen neue, die markierte Sonde enthaltende Hybridisierungslösung ausgetauscht. Vor Zugabe der Sonde wurde diese für 10 min im Wasserbad (100°C) denaturiert und anschließend für 5 min auf Eis inkubiert. Je ml Hybridisierungslösung wurden 2-5 µl markierte Sonden-DNA eingesetzt. Hybridisiert wurde für mindestens 15 h bei 39°C. Die Detektierungsreaktion erfolgte nach Protokolle der Fa. Roche. Die Exposition der Röntgenfilme betrug, in Abhängigkeit von der Signalstärke, zwischen 10 min und 2 h. Die Rehybridisierung der Filter erfolgte nach Angaben des Herstellers. Die Sondenlösungen wurden bei -20°C gelagert und nach Denaturierung bei 68°C im Wasserbad für weitere 8 bis 10 Hybridisierungen verwendet.

### Beispiel 4:

### Nachweis mt-spezifischer Signale nach Hybridisierung von Gesamt-DNA

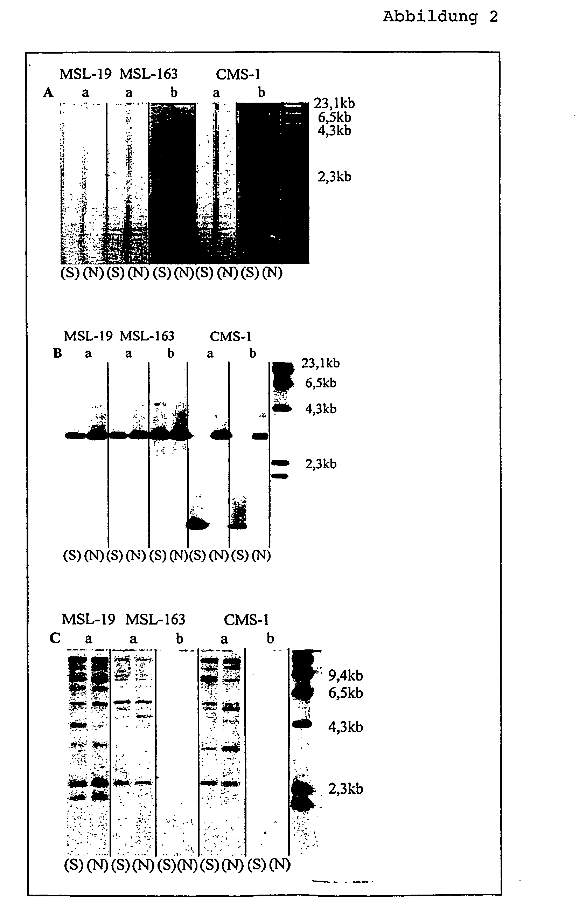

Zunächst wurde geklärt, ob die Verwendung von Gesamt-DNA in Verbindung mit den mitochondrialen Sonden nicht zu anderen oder zusätzlichen Hybridisierungssignalen führen kann, welche auf das Vorhandensein mitochondrialer Sequenzen im Kerngenom zurückzuführen sein könnten. Aus diesem Grund wurde aus frisch geernteten Blattproben der zu untersuchenden Pflanzen CMS-1 (N), CMS-1 (S), MSL-163 (N) und MSL-163 (S) jeweils mitochondriale DNA isoliert und die Hybridisierungsergebnisse mit denen unter Verwendung von Gesamt-DNA verglichen. Nach Restriktion von Gesamt- und mtDNA der gleichen Linie mit dem Restriktionsenzym *Hind*III und elektrophoretischer Auftrennung der Proben im Agarosegel konnte nach Ethidiumbromidfärbung visuell keine DNA nachgewiesen werden. In den Spuren mit Gesamt-DNA war eine kontinuierliche Fragmentverteilung im Größenbereich von ca. 1 bis 23 kb zu beobachten, was auf eine vollständige Restriktion der DNA hinweist (siehe Abbildung 2A).

Nach Hybridisierung mit der mt-Gensonde *nad9* und mtDNA bzw. Gesamt-DNA als Template konnten identische Hybridisierungssignale nachgewiesen werden. Das Ergebnis zeigt, dass unter Verwendung von mt-Gensonden und Gesamt-DNA als Template mtDNA-spezifische Signale nachgewiesen werden können (siehe Abbildung 2B).

Im Vergleich dazu konnte unter Verwendung einer cDNA-Sonde des kernkodierten Actingens gezeigt werden, dass keine Kontamination der mtDNA mit genomischer DNA vorliegt, da wie erwartet nur in den Spuren mit Gesamt-DNA Hybridisierungssignale nachzuweisen waren (siehe Abbildung 2C). Da somit die mtDNA-Spezifität der Sonden gegeben war, wurde im weiteren Gesamt-DNA verwendet.

Für die eingehenden molekularbiologischen Untersuchungen wurde das Pflanzenmaterial in zwei Gruppen eingeteilt. Die erste Gruppe bestand aus der CMS-Vergleichslinie CMS-1 (S) und dem zugehörigen Maintainer CMS-1 (N), die zweite Gruppe beinhaltete die männlich sterilen MSL-Linien MSL-163 (S) und MSL-19 (S) und zugehörige männlich fertile Maintainerlinien MSL-163 (N) und MSL-19 (N).

Insgesamt wurden 34 verschiedene Sonden/Enzym-Kombinationen getestet, von denen 14 eine Unterscheidung der männlich sterilen Linien (S) zu den jeweils zugehörigen Maintainem (N) möglich machten (siehe Tabelle 9).

Des Weiteren konnten die (S)-Plasmen der beiden Gruppen CMS und MSL insbesondere unter Verwendung der Sonde *nad9* in Kombination mit verschiedenen Restriktionsenzymen eindeutig differenziert werden (siehe Tabelle 10).

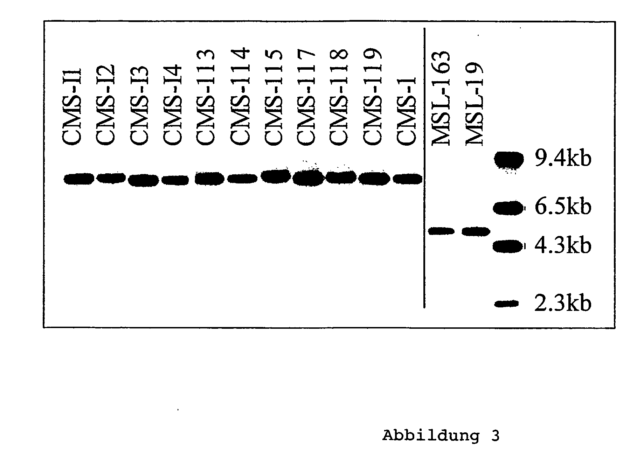

Mit der Sonden/Enzym-Kombination *nad9*/*Dra*I wurde anschließend ein erweitertes Set von verfügbaren *Lolium*-CMS-Quellen in die Untersuchungen integriert: drei Pflanzen der CMS-Inca Quelle (CMS-I1, CMS-I2, CMS-I3, CMS-I4), CMS-113, CMS-114, CMS-115, CMS-117, CMS-118 und CMS-119 (siehe Abbildung 3). Hierbei konnten die MSL-Pflanzen MSL-163 und MSL-19 eindeutig von allen anderen CMS-Quellen unterschieden werden, die ihrerseits untereinander ein identisches Bandenmuster aufwiesen.

### Abbildungen

Abbildung 1: A, Pollen einer Einzelpflanze CMS-1 (S) nach KES-Färbung; B, Pollen einer Einzelpflanze CMS-1 (N) nach KES-Färbung (Vergrößerungsmaßstäbe nicht identisch).
Abbildung 2:A, Resbriktionsverdau von Gesamt-DNA (a) und mtDNA (b) der Pflanzen MSL-19, MSL-163 und CMS-1 mit dem Restriktionsenzym *Hind*III; B, Southern-Hybridisierung mit der Sonden/Enzymkombination *nad9*/*Hind*III; C, Southern-Hybridisierung mit der Sonden/Enzymkombination *Actin*/*Hind*III; (S) = CMS-Pflanze; (N) = Maintainer.
Abbildung 3: Southern-Hybridisierung (Sonden/Enzymkombination *nad9*/*Dra*I) von DNA-Bulks der Pflanze Inca (CMS-I1, CMS-I2, CMS-I3, CMS-I4) und der CMS-1 (S), MSL-163 (S) und MSL-19 (S) und von Einzelpflanzen der Herkünfte CMS-113, CMS-114, CMS-115, CMS-117, CMS-118 und CMS-119.

**Tabelle 1: Färbemethoden zum Pollen-Vitalitätstest**

| **Methode zur Differenzierung von fertilem und sterilem Pollen** | **(ALEXANDER, 1969)** |
|---|---|
| Färbung: | |
| fertiler Pollenkörner | dunkel lila |
| steriler Pollenkörner | Hellgrün |
| Zusammensetzung der Lösung: | |
| Äthylalkohol | 10ml |
| Malachitgrün (1% in 96% Alkohol) | 1ml |
| dest. Wasser | 50ml |
| Glycerin | 25ml |
| Phenol | 5g |
| Chloralhydrat | 5g |
| Fuchsin (1% in Wasser) | 5ml |
| Orange G (1% in Wasser) | 0,5ml |
| Eisessig (pH 3,2) | 1-2ml |
| | |
| **Lichtgrün** | **(** 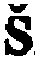 **INSKA, 1976)** |
| Färbung: | |
| fertile Pollenkörner | Tiefgrün mit deutlich netzartiger Oberflächenstruktur |
| sterile Pollenkörner | meist völlig farblos und durch degeneriertes Plasma schwach grün gefärbt |
| Zusammensetzung der Lösung: | |
| Glycerin | 1 Teil |
| Milchsäure | 1 Teil |
| Phenol | 1 Teil |
| | |
| **Lugolsche Lösung** | |
| Färbung: | |
| Fertile Pollenkörner | Rotfärbung |
| Zusammensetzung der Lösung: | |
| Jod-Jod-Kalium | |

**Tabelle 2: Merkmalsausprägungen von Kreuzungsnachkommen der CMS-Linie MSL-19 und CMS-1 (beide Lolium perenne)**

| **Merkmal** | **MSL-19** | **CMS-1** |
|---|---|---|
| Ploidiestufe | diploid | diploid |
| Entstehung | experimentelle Mutagenese mit N-Ethylharnstoff | Spontan (1969 u. 1970 in Sortimenten u. Vermehrungsbeständen in Gülzow gefunden; BURKERT u. SCHLENKER, 1975) |
| Visuelle Sterilität | sehr gut | Sehr gut |
| Antherenausprägung | weiß-grün-gelbsteril bzw. Mischfarben | Weiß-grün-gelbsteril Bzw. Mischfarben |
| Visuelle Sterilität/ Sterilitätsgrad | gute Übereinstimmung unabhängig von der Antherenausprägung | Keine Übereinstimmung hoher Anteil Teilsteriler in Abhängigkeit von der Antherenausprägung |
| Umweltabhängigkeit | sehr niedrig | Hoch |

**Tabelle 3: Anteil vollsteriler Genotypen in F₁-Kreuzungsnachkommenschaften bei Lolium perenne in Abhängigkeit von der CMS-Quelle**

| **CMS-Quelle (S)** | **Entstehungsweise** | **Anzahl untersuchter visuell steriler F₁-Pflanzen nach Rückkreuzung mit Maintainer (N)** | **relativer Anteil vollsteriler Pflanzen** |
|---|---|---|---|
| CMS-1 | spontan | 1333 | 53,3^{x)} |
| CMS-INCA | Interspezifische Kreuzung | 172 | 47,7^{x)} |
| CMS-5 B (Irland) | Interspezifische Kreuzung | 225 | 77,0^{xx)} |
| MSL-19 | Mutagenese mit N-Ethylharnstoff | 1500 | 99,0^{xxx)} |

| | | | |
|---|---|---|---|
| ^{x)} Nachweis Färbemethode Lichtgrünreagenz ^{xx)} nach Conolly (1984) Sterilitätstyp 1 und 2^{v} ^{xxx)} Nachweis Färbemethode Alexander ^{v} Sterilitätstyp 1: Antheren sind flach, platzen nicht auf, sind zusammengeschrumpft, meist weiß oder durchsichtig mit dünnen Zellwänden. Die Antheren enthalten keinen Pollen. Sterilitätstyp 2: Antheren platzen nicht auf und zusammengeschrumpft, jedoch nicht so flach wie 1, Antheren enthalten keinen lebensfähigen Pollen, leere Pollen sind z.T. sehr schwach gefärbt. | | | |

**Tabelle 4: Sterilitätsvererbung der Lolium perenne CMS-Linien MSL-19 und CMS-1; Ergebnisse der Sterilitätsüberprüfung an F₁-Material mit identischen Bestäubem (Maintainern)**

| **Kreuzungskombination CMS (S) x Bestäuber (N)** | **Anzahl untersuchte F₁-Pflanzen** | **Ergebnisse Sterilitätsprüfung (Relativwerte)** | |
|---|---|---|---|
| | | **visuelle Sterilität** | **Pollenvitalität^{x}** |
| MSL-19x 85/5/9/6 | 30 | 100 | 0 |
| MSL-19x KE 23/85 | 35 | 100 | 0 |
| CMS- x 85/5/9/6 | 30 | 100 | 15,20 |
| CMS-1 x KE 23/85 | 30 | 100 | 13,80 |

| | | | |
|---|---|---|---|
| *nach Färbemethode von Alexander (siehe oben) | | | |

**Tabelle 5: Funktion der verwendeten mitochondrialen Gene bei höheren Pflanzen**

| **Protein** | **Mitochondriales Gen** |
|---|---|
| Untereinheiten der Cytochrom C-Oxidase | *coxI, coxII, coxIII* |
| NADH-Dehydrogenase | *nad6* |
| NADH: Ubichinon-Oxidoreduktase | *nad9* |
| Ribosomale Proteine: große Untereinheit Kleine Untereinheit | *rpl6 rps3* |
| Apocytochrom b | *cob* |
| Cytochrom C Biogenese ORF 206 | *ccb206* |

**Tabelle 6: Beschreibung der verwendeten Primerpaare (U = oberer Primer, L = unterer Primer)**

| Sonde | **Primer (5'- 3')** | | **Annealing-temperatur** | **Amplicon (bp)** |
|---|---|---|---|---|
| *coxI* | TTACTTCACATAGCTTTTCGTU | U | 52.1°C | 1556 |
| | CCACAAACCACAAGGATATAG | L | | |
| *coxII* | CGTAAAGGCATGATTAGTTCC | U | 52.3°C | 697 |
| | GATTGTTCTAAAATGGTTATTCCTC | L | | |
| *coxIII* | ATGATTGAATCTCAGAGGCAT | U | 53.0°C | 797 |
| | CATATACCTCCCCACCAATAG | L | | |
| *nad6* | TTAGTAGATCGTGAGTGGGTC | U | 51.6°C | 563 |
| | GTGCTAAAAATCCGGTACAT | L | | |
| *nad9* | TTATCCGTCGCTACGCTGTTC | U | 55.0°C | 3392 |
| | AATGGAAAGATCGGAACATGG | L | | |
| *rpl5* | ATGTTTCCACTCAATTTTCAT | U | 52.2°C | 522 |
| | GCTCCACAGTGGTAAAGTCT | L | | |
| *rpl6* | TTACGACCACTGAACAAACTT | U | 53.0°C | 535 |
| | TTTAACCATAAAATCGATTATGC | L | | |
| *rps3* | CTATATTTCGTACGTTTCGGA | U | 52.4°C | 1594 |
| | TTATTATGGTAAATTTGTGTATCAA | L | | |
| *cob* | ATGACTATAAGGAACCAACGA | U | 52.1°C | 1174 |
| | GATCAGTCTCATCCGTGTAA | L | | |
| *ccb20* | ATGAGACGACTTTTTCTTGAA | U | 52.2°C | 616 |
| *6* | CTTGTAAACTAATCGAGACCG | L | | |
| Actin | CACACTGTCCCCATCTATGAA | U | 57.9°C | 650 |
| | CTCTTGGCTTAGCATTCTTGG | L | | |

**Tabelle 7: PCR-Bedingungen zur Erzeugung der genutzten DNA-Sonden**

| **PCR-Komponenten** | **Menge** |
|---|---|
| DIG-dUTP (10x) | 5µl |
| d-NTP-Mix (10mM) | 1 µl (200µM) |
| Primer (5µM) | Je 2,5µl (je 0,25µM) |
| Taq-Polymerase* (5U/µl) | 0,15µl (0,75 U) |
| 10x-Reaktionspuffer | 5µl |
| MgCl₂-Lösung (50mM) | 1,55µl (1,5µM) |
| H₂O | 17,3µl |
| Template-DNA | 15µl (75ng) |
| | Reaktionsvolumen 50µl |

| | |
|---|---|
| *Silverstar, Fa. Eurogentec | |

**Tabelle 8: PCR-Bedingungen (30 Zyklen 2.-4.)**

| | | | |
|---|---|---|---|
| 1. | Denaturierung (einmalig) | 94°C | 2min |
| 2. | Denaturierung | 94°C | 1min |
| 3. | Annealing | s. Tab. 6 | 1min |
| 4. | Extension | 72°C | 2min |
| 5. | Extensionsverlängerung (einmalig) | 72°C | 2min |

**Tabelle 9: Differenzierung zwischen (S)- und (N)-Cytoplasma der Pflanzen MSL-19 und MSL-163 (MSL) im Vergleich zur Pflanze CMS-1 (CMS)**

| | **Restriktionsenzym** | | | | | | | | | | | |
|---|---|---|---|---|---|---|---|---|---|---|---|---|
| **mt-** | *Hind*III | | *Xba*I | | *Dra*I | | *Eco*RV | | *Bam*HI | | *Hae*III | |
| **Gen-** | MSL | | MSL | | MSL | | MSL | | MSL | | MSL | |
| **sonde** | CMS | | CMS | | CMS | | CMS | | CMS | | CMS | |
| *coxI* | + | + | n. g. | | + | + | n. g. | | + | + | n. g. | |
| *coxIII* | + | + | n. g. | | + | + | + | + | + | + | n. g. | |
| *nad6* | + | + | n. g. | | + | + | - | - | + | + | - | - |
| *nad9* | - | + | - | + | + | + | - | + | + | + | - | + |
| *ccb206* | - | - | - | - | - | - | + | - | - | - | - | - |
| *rpl5* | - | - | n. g. | | n. g. | | - | - | n. g. | | n. g. | |
| *rpl6* | + | - | - | - | + | + | n. g. | | + | - | n. g. | |
| *cob* | n. g. | | + | - | n. g. | | + | - | n. g. | | + | - |
| *rps3* | n. g. | | n. g. | | n. g. | | + | + | n. g. | | n. g. | |

| | | | | | | | | | | | | |
|---|---|---|---|---|---|---|---|---|---|---|---|---|
| MSL = DNA Bulk der Pflanzen MSL-19 und MSL-163 CMS = männlich sterile Pflanze CMS-1 + = Differenzierung zwischen (N)- und (S)-Cytoplasma möglich - = Differenzierung zwischen (N)- und (S)-Cytoplasma nicht möglich n. g. = nicht getestete Sonden-/Enzymkombinationen | | | | | | | | | | | | |

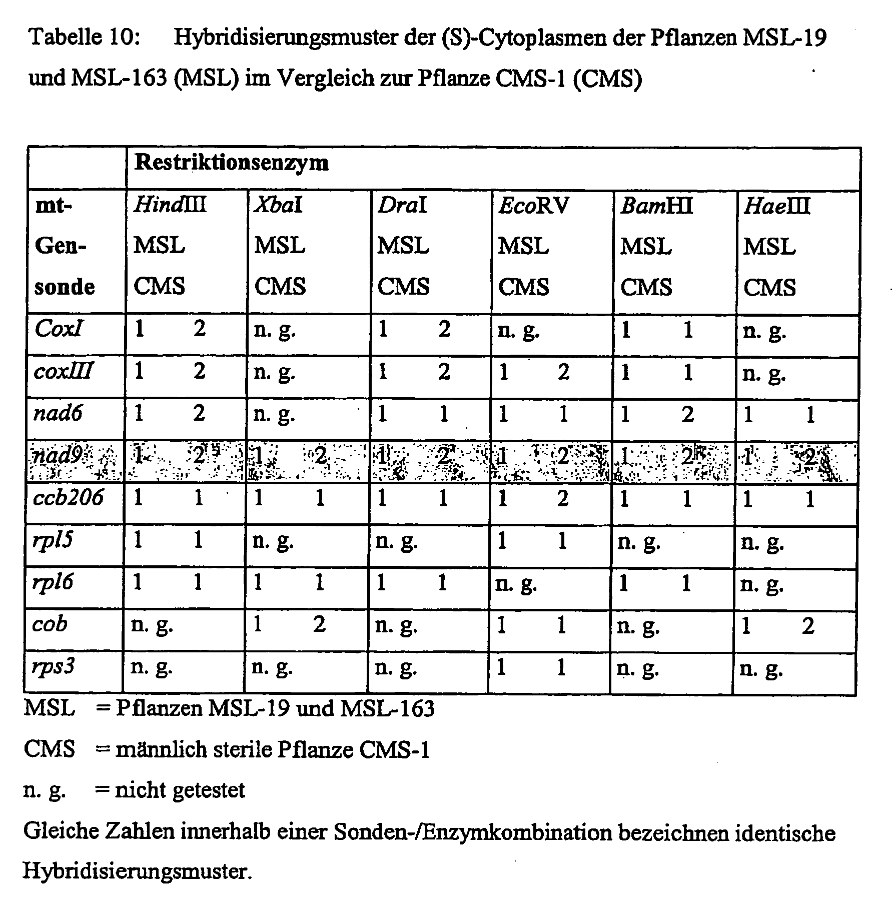

### Anhang A

### Isolation von Gesamt-DNA:

| **2xCTAB** | **TE-Puffer** |
|---|---|
| 0.1 M Tris/HCl pH 8.0 | 10 mM Tns/HCl pH 8.0 |
| 1.4 M NaCl | 1 mM EDTA pH 8.0 |
| 0.5 M EDTA pH 8.0 | |
| 2% CTAB | |
| 0.04 M β-Mercaptoethanol | |

### Isolation von mtDNA

| **Extraktionspuffer 1** | **DNase-Puffer** |
|---|---|
| 0.44 M Sucrose | 0.44 M Sucrose |
| 50 mM Tris pH 8.0 | 50 mM Tris pH 8.0 |
| 3 mM EDTA pH 8.0 | 10 mM MgCl₂ |
| 0.5% BSA | |
| 10 mM β-Mercaptoethanol | |

| **Waschpuffer** | **Lysispuffer** |
|---|---|
| 0.6 M Sucrose | 50 mM Tris |
| 25 mM EDTA pH 8.0 | 20 mM EDTA |
| 50 mM Tris pH 8.0 | 0.5% SDS |
| | pH 8.0 |

| **Extraktionspuffer** 2 | **"50-10" TE-Puffer** |
|---|---|
| 0.15 M Tris | 50 mM Tris |
| 0.1 M NaCl | 10 mM EDTA |
| 80 mM EDTA | pH 8.0 |
| 1.5% SDS | |
| pH 8.0 | |

| **TE-Puffer** |
|---|
| 10 mM TRIS |
| 1 mM EDTA |
| pH 8.0 |

### Elektrophorese und Blotting

| | |
|---|---|
| 1 x TAE | 20x SSC |
| 40 mM Tris/Acetat | 3 M NaCl |
| 1 mM EDTA | 0.3 M Na-Citrat |
| | pH 7.0 |

### 5x Ladepuffer

12.5 % Ficoll
62.5 mM EDTA pH 8.0
0.5 % SDS
5x TAE
0.02 % Bromphenol Blau
0.02 % Xylene Cyanol

## Patentansprüche

1. Verfahren zur Herstellung von vollständig männlich sterilen Pflanzen der Gattung Lolium, umfassend die folgenden Schritte:
a) Mutagenese von Karyopsenmaterial von Wildtyppflanzen der Gattung Lolium;
b) Untersuchung der mutagenisierten Lolium-Pflanzen mit Southern-Blot-Techniken; und
c) Identifizierung von vollständig männlich sterilen Lolium-Pflanzen.
**dadurch gekennzeichnet, dass** die Primerpaare zur Amplifizierung der bei der Southern-Blot-Hybridisierung eingesetzten Sonden aus der Gruppe, bestehend aus den folgenden Primerpaaren ausgewählt werden:
a) TTACTTCACATAGCTTTTCGTU
CCACAAACCACAAGGATATAG
b) ATGATTGAATCTCAGAGGCAT
CATATACCTCCCCACCAATAG
c) TTAGTAGATCGTGAGTGGGTC
GTGCTAAAAATCCGGTACAT
d) TTATCCGTCGCTACGCTGTTC
AATGGAAAGATCGGAACATGG
e) ATGACTATAAGGAACCAACGA
GATCAGTCTCATCCGTGTAA
f) ATGAGACGACTTTTTCTTGAA
CTTGTAAACTAATCGAGACCG,
wobei die Sequenzen in 5'-3'-Richtung angegeben sind und jeweils die erste Sequenz den oberen Primer darstellt,
und die durch die Amplifikation mit den Primern hergestellten Sonden in einer der folgenden Kombinationen mit Restriktionsenzymen zur Southern-Blot-Analyse eingesetzt werden:
a) zusammen mit HindIII oder DraI
b) zusammen mit HindIII, DraI oder EcoRV
c) zusammen mit HindIII oder BamHI
d) zusammen mit HindIII, XbaI, DraI, EcoRV, BamHI oder HaeIII
e) zusammen mit XbaI oder HaeIII
f) zusammen mit EcoRV.

2. Verfahren nach Anspruch 1,
**dadurch gekennzeichnet, dass** Mutagenese durch Zugabe von chemischen Mutagenen, insbesondere von N-Ethylharnstoff erfolgt.

3. Verfahren nach Anspruch 1 oder 2,
**dadurch gekennzeichnet, dass** die Lolium-Pflanzen ausgewählt werden aus der Gruppe bestehend aus Lolium perenne, Lolium multiflorum und Lolium hybridum.

4. Verfahren zur Unterscheidung von MSL-Pflanzen von Lolium-Pflanzen mit teilweiser oder instabiler männlicher Sterilität durch Southern-Blot-Hybridisierung, wobei die Primerpaare zur Amplifizierung der bei der Southern-Blot-Hybridisierung eingesetzten Sonden aus der Gruppe, bestehend aus den folgenden Primerpaaren ausgewählt werden:
a) TTACTTCACATAGCTTTTCGTU
CCACAAACCACAAGGATATAG
b) ATGATTGAATCTCAGAGGCAT
CATATACCTCCCCACCAATAG
c) TTAGTAGATCGTGAGTGGGTC
GTGCTAAAAATCCGGTACAT
d) TTATCCGTCGCTACGCTGTTC
AATGGAAAGATCGGAACATGG
e) ATGACTATAAGGAACCAACGA
GATCAGTCTCATCCGTGTAA
f) ATGAGACGACTTTTTTCTTGAA
CTTGTAAACTAATCGAGACCG,
wobei die Sequenzen in 5'-3'-Richtung angegeben sind und jeweils die erste Sequenz den oberen Primer darstellt,
und die durch die Amplifikation mit den Primern hergestellten Sonden in einer der folgenden Kombinationen mit Restriktionsenzymen zur Southern-Blot-Analyse eingesetzt werden:
a) zusammen mit HindIII oder DraI
b) zusammen mit HindIII, DraI oder EcoRV
c) zusammen mit HindIII oder BamHI
d) zusammen mit HindIII, XbaI, DraI, EcoRV, BamHI oder HaeIII
e) zusammen mit XbaI oder HaeIII
f) zusammen mit EcoRV.

## Claims

1. Process for the production of completely male sterile plants of the genus *Lolium* comprising the steps of:
a) mutagenesis of caryoptic material of wild type plants of the genus *Lolium;*
b) analysis of the mutagenized *Lolium* plants by Southern Blot techniques; and
c) identification of completely male sterile *Lolium* plants;
**characterized by** the fact that the pairs of primers for amplification of the probes employed during Southern Blot hybridisation are selected from the group consisting of:
a) TTACTTCACATAGCTTTTCGTU
CCACAAACCACAAGGATATAG
b) ATGATTGAATCTCAGAGGCAT
CATATACCTCCCCACCAATAG
c) TTAGTAGATCGTGAGTGGGTC
GTGCTAAAAATCCGGTACAT
d) TTATCCGTCGCTACGCTGTTC
AATGGAAAGATCGGAACATGG
e) ATGACTATAAGGAACCAACGA
GATCAGTCTCATCCGTGTAA
f) ATGAGACGACTTTTTCTTGAA
CTTGTAAACTAATCGAGACCG,
wherein the sequences are given in the 5'-3' direction and each of the first sequences represents the upper primer; and
the probes produced by amplification with the primers are employed in one of the following combinations with restriction enzymes for Southern Blot analysis:
a) together with HindIII or DraI
b) together with HindIII, Dral or EcoRV
c) together with HindIII or BamHI
d) together with HindIII, XbaI, DraI, EcoRV, BamHI or HaeIII
e) together with XbaI or HaeIII
f) together with EcoRV.

2. Process according to claim 1,
**characterized by** the fact that mutagenesis is done by the addition of chemical mutagens, particularly N-ethyl urea.

3. Process according to claim 1 or 2,
**characterized by** the fact that the *Lolium* plants are selected from the group consisting of *Lolium perenne, Lolium multiflorum* and *Lolium hybridum.*

4. Process for the distinction of MSL plants from *Lolium* plants having partial or unstable male sterility by Southern Blot hybridisation wherein the pairs of primers for amplification of the probes employed during Southern Blot hybridisation are selected from the group consisting of the following pairs of primers:
a) TTACTTCACATAGCTTTTCGTU
CCACAAACCACAAGGATATAG
b) ATGATTGAATCTCAGAGGCAT
CATATACCTCCCCACCAATAG
c) TTAGTAGATCGTGAGTGGGTC
GTGCTAAAAATCCGGTACAT
d) TTATCCGTCGCTACGCTGTTC
AATGGAAAGATCGGAACATGG
e) ATGACTATAAGGAACCAACGA
GATCAGTCTCATCCGTGTAA
f) ATGAGACGACTTTTTCTTGAA
CTTGTAAACTAATCGAGACCG,
wherein the sequences are given in the 5'-3' direction and each of the first sequences represents the upper primer; and
the probes produced by amplification with the primers are employed in one of the following combinations with restriction enzymes for Southern Blot analysis:
a) together with HindIII or DraI
b) together with HindIII, DraI or EcoRV
c) together with HindIII or BamHI
d) together with HindIII, XbaI, DraI, EcoRV, BamHI or HaeIII
e) together with XbaI or HaeIII
f) together with EcoRV.

## Revendications

1. Procédé pour la production des plants complètement mâles-stériles d'espèce *Lolium* comprenant les étapes suivantes :
a) mutagénèse de matériel caryopse de plants de type sauvage d'espèce *Lolium* ;
b) analyse des plants Lolium mutagenisées par des techniques de Southern Blot ; et
c) identification des plants *Lolium* complètement mâles-stériles,
**caractérisé par le fait que** les paires d'amorces pour l'amplification des sondes mises en oeuvre chez l'hybridation de Southern Blot sont sélectionnées parmi le group consistant en :
a) TTACTTCACATAGCTTTTCGTU
CCACAAACCACAAGGATATAG
b) ATGATTGAATCTCAGAGGCAT
CATATACCTCCCCACCAATAG
c) TTAGTAGATCGTGAGTGGGTC
GTGCTAAAAATCCGGTACAT
d) TTATCCGTCGCTACGCTGTTC
AATGGAAAGATCGGAACATGG
e) ATGACTATAAGGAACCAACGA
GATCAGTCTCATCCGTGTAA
f) ATGAGACGACTTTTTCTTGAA
CTTGTAAACTAATCGAGACCG,
dans lequel les séquences sont indiquées en direction 5'-3' et toutes les premières séquences représentent l'amorce supérieure,
et les sondes fabriquées par l'amplification avec les amorces sont mises en oeuvre dans une des associations suivantes avec des enzymes de restriction pour l'analyse de Southern Blot :
a) en association avec HindIII ou DraI
b) en association avec HindIII, DraI ou EcoRV
c) en association avec HindIII ou BamHI
d) en association avec HindIII, XbaI, DraI, EcoRV, BamHI ou HaeIII
e) en association avec XbaI ou HaeIII
f) en association avec EcoRV.

2. Procédé selon la revendication 1,
**caractérisé par le fait que** la mutagénèse aura lieu par l'addition de mutagènes chimiques, particulièrement de la N-éthyle urée.

3. Procédé selon la revendication 1 ou 2,
**caractérisé par le fait que** les plants *Lolium* sont sélectionnées parmi le group consistant en *Lolium perenne, Lolium multiflorum* et *Lolium hybridum.*

4. Procédé pour la distinction des plants MSL et des plants *Lolium* ayant une stérilité mâle partielle ou instable par l'hybridation de Southern Blot, dans lequel les paires d'amorces pour l'amplification des sondes mises en oeuvre chez l'hybridation de Southern Blot sont sélectionnées parmi le group consistant en les paires d'amorces suivantes :
a) TTACTTCACATAGCTTTTCGTU
CCACAAACCACAAGGATATAG
b) ATGATTGAATCTCAGAGGCAT
CATATACCTCCCCACCAATAG
c) TTAGTAGATCGTGAGTGGGTC
GTGCTAAAAATCCGGTACAT
d) TTATCCGTCGCTACGCTGTTC
AATGGAAAGATCGGAACATGG
e) ATGACTATAAGGAACCAACGA
GATCAGTCTCATCCGTGTAA
f) ATGAGACGACTTTTTCTTGAA
CTTGTAAACTAATCGAGACCG,
dans lequel les séquences sont indiquées en direction 5'-3' et toutes les premières séquences représentent l'amorce supérieure,
et les sondes fabriquées par l'amplification avec les amorces sont mises en oeuvre dans une des associations suivantes avec des enzymes de restriction pour l'analyse de Southern Blot :
a) en association avec HindIII ou DraI
b) en association avec HindIII, DraI ou EcoRV
c) en association avec HindIII ou BamHI
d) en association avec HindIII, XbaI, DraI, EcoRV, BamHI ou HaeIII
e) en association avec XbaI ou HaeIII
f) en association avec EcoRV.
